(19) Europäisches Patentamt
European Patent Office
Office européen des brevets

(11) Publication number: **0 491 472 A2**

# (12) EUROPEAN PATENT APPLICATION

(21) Application number: **91310780.1**

(51) Int. Cl.5: **A61K 7/06**, A61K 7/48

(22) Date of filing: **22.11.91**

(30) Priority: **18.12.90 JP 411371/90**

(43) Date of publication of application: **24.06.92 Bulletin 92/26**

(84) Designated Contracting States: **CH DE GB LI NL**

(71) Applicant: **KAO CORPORATION**
**1-14-10, Nihonbashikayaba-cho**
**Chuo-ku Tokyo(JP)**

(72) Inventor: **Hikichi, Tadasu**
**7840-1 Yuki**
**Yuki-shi, Ibaraki(JP)**
Inventor: **Aihara, Kayoko**
**2-9-28 Nakakasai, Edogawa-ku**
**Tokyo(JP)**

(74) Representative: **Daley, Michael John et al**
**F.J. CLEVELAND & COMPANY 40/43**
**Chancery Lane**
**London, WC2A 1JO(GB)**

## (54) Hair cosmetic composition in gel state.

(57) A hair cosmetic composition in a gel state, which comprises the following ingredients (a), (b), (c), (d) and (e):
(a) a salt of a monoalkyl phosphate of formula (1):

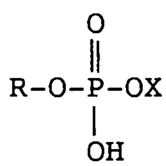

(1)

wherein R is a beta-branched alkyl having 8 to 36 carbon atoms, X is an alkali metal, basic amino acid or an organic base;
(b) water;
(c) a volatile oil;
(d) a non-volatile oil; and
(e) an alcohol.

The composition has excellent extendibility, free of greasiness when applied to the hair, and provides agreeable feeling to the tough to the hair when the hair applied with the composition is dried.

EP 0 491 472 A2

This invention relates to a hair cosmetic composition in the gel state and sets out to provide a hair cosmetic composition in a gel state which has a good extendibility on hands and the hair for securing easy application to the hair and imparts an agreeable feel to the hair upon application.

Several methods are known for obtaining a hair cosmetic composition in a gel state which include:-

(1) A combination of a nonionic surfactant/water/polyol is added with an oil (Jpn. pat. appln. Kokai No. 61-37709),

(2) A water-soluble polymer such as carboxyvinyl polymer is employed, and

(3) A fatty acid soap, dextrin fatty ester, etc. are used to make an oil into a gel.

However, these methods are accompanied by drawbacks respectively: In method (1), the stability of the combination of a nonionic surfactant/water/polyol is affected by the ambient temperature, which means unreliable temperature stability, and besides, oils to be incorporated are limited. In method (2), the slimy touch inherent to water-soluble polymers presents disagreeable feeling upon use, and moreover, since the polymers make the viscosity of the composition increased, spinnability of the composition brings about disagreeable extendibility upon use. In method (3), oil is solely turned into a gel state, and the resulting composition is sticky to hands and the hair, while the stickiness should desirably be avoided.

Accordingly, a hair cosmetic composition in a gel state which has a good extendibility on hands and the hair, is free from oiliness and has agreeable feeling upon use is still desired.

The present inventors have made careful studies and have found that a specific combination of a surfactant, water, an alcohol, a non-volatile oil and a volatile oil, where the surfactant is a salt of a monoalkyl phosphate having a specified branching, provides an excellent hair cosmetic composition in a gel state, which extends smoothly on hands and the hair, is free from greasiness when applied to the hair, and further presents agreeable touch to the hair with innocence of greasiness when the hair is dried. The present invention was accomplished based on this finding.

Accordingly, an object of this invention is to provide a hair cosmetic composition in a gel state, which comprises the following ingredients (a), (b), (c), (d) and (e):

(a) a salt of a monoalkyl phosphate of formula (1):

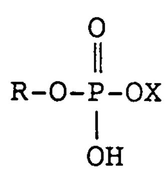

(1)

wherein R is a beta-branched alkyl having 8 to 36 carbon atoms, X is an alkali metal, basic amino acid or an organic base;

(b) water;

(c) a volatile oil;

(d) a non-volatile oil; and

(e) an alcohol.

The salt (1) of monoalkylphosphate which is useful as ingredient (a) in this invention has a branching at the beta- position of its alkyl group. Exemplary process for obtaining this is disclosed for example in Japanese patent application kokai Nos. 58-180496 and 61-17594. Among various salts (1), it is preferred to use a compound where R in formula (1) is a beta-branched alkyl defined as follows:

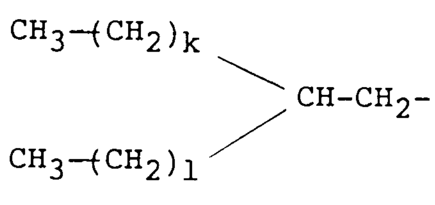

(2)

wherein k denotes a number of 2 - 18, 1 denotes a number of 2 - 14 provided k plus 1 is from 4 to 32, preferably 10 to 18. Examples of alkali metal usable as the counter ion X include lithium, potassium and sodium; the basic amino acids include arginine, ornithine, lysine and oxylysine; the organic bases include alkanolamines having a hydroxyalkyl group of C2-C3 such as triethanolamine and monoethanolamine.

In this invention, both volatile and non-volatile oils (c) and (d) are used. The definitions of "volatile " and

"non-volatile" are given as follows: A filter paper is permeated with a sample, and then allowed to stand at 30 °C. The value of 50% by weight for the amount volatiled in 3 hours separates "volatile oil" (less than 50% volatility) and "non-volatile oil" (50% or more volatility).

Examples of the volatile oils (c) which are useful in this invention include volatile linear silicones, volatile cyclic silicones and volatile hydrocarbons. Exemplary volatile linear silicones are dimethylpolysiloxanes represented by the following formula (3):

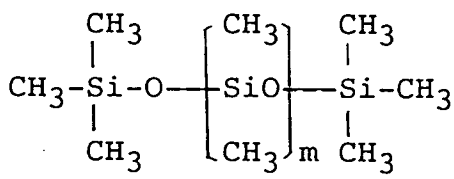

(3)

wherein m denotes a number of 0 to 5. In particular, the following are mentioned: hexamethyl disiloxane, octamethyl trisiloxane, decamethyl tetrasiloxane, hexadecamethyl heptasiloxane. Exemplary volatile cyclic silicones are those represented by the following formula (4):

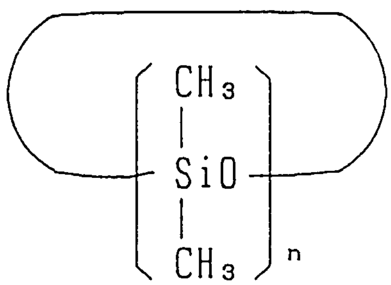

(4)

wherein n denotes a number of 3 to 7. In particular, the following are mentioned: octamethyl cyclotetrasiloxane, decamethyl cyclopentasiloxane, tetradecamethyl cyclohexasiloxane. Exemplary volatile hydrocarbons are linear or branched hydrocarbons of C10-C16, preferably C12-C16. In particular, mention may be given to NISSEKI ISOZOL 300, NISSEKI ISOZOL 400 (both products of Nihon Sekiyu), IP SOLVENT 1016, IP SOLVENT 1620 (both products of IDEMITSU SEKIYU), NAS-3, NAS-4, PARLEAM 4 (all products of NIPPON YUSHI).

Non-volatile oils (d) which are useful in this invention are hydrocarbons, higher alcohols, higher fatty acids, higher fatty acid esters, oils and fats of animal or vegetable origin, cholesterol fatty acid esters, higher fatty acid glyceryl esters, rosin derivatives and silicone oils, so long as they are determined as "non-volatile" according to the volatility test mentioned before. Among them, particularly preferred are diglyceryl esters of one or more fatty acids such as isostearyl cholesteryl ester, 2-ethylhexanoic acid, myristic acid, oleic acid and isostearic acid; triglyceryl esters of one or more fatty acids such as 2-ethylhexanoic acid, myristic acid, oleic acid and isostearic acid; octyldodecyl myristate, liquid paraffin, squalane, di-2-ethylhexanoic neopentyl glycol, non-volatile dimethylpolysiloxane and isopropyl myristate. These non-volatile oils are used singly or as a mixture.

Examples of alcohols (e) include monoalcohols such as ethanol, and polyols such as glycerol, ethylene glycol, diethylene glycol, triethylene glycol, hexanediol, butylene glycol, heptanediol, propylene glycol, sorbitol and maltitol, among which ethanol, glycerol, propylene glycol and sorbitol are particularly preferred.

Preferable proportions of the ingredients (a) to (e) are as follows:

(a): 0.05 - 5% by weight, in particular 0.1 - 2% by weight,
(b): 0.5 - 50% by weight, in particular, 2 - 30% by weight,
(c): 50 - 98% by weight,
(d): 0.1 - 20% by weight, in particular, 0.5 - 10% by weight,
(e): 2- 50% by weight, in particular, 3 - 40% by weight.

The hair cosmetic composition in a gel state according to this invention may optionally incorporate other compatible ingredients which are generally used in this technical field. They are moisturizers, blood circulation promoters, cool-feeling agents, UV absorbers, antiperspirants, germicides, skin activators, anti-aging agents, perfumes, colorants, and so on.

The hair cosmetic composition in a gel state of this invention is prepared according to a conventional method, and preferably, ingredients (a), (b) and (e) are first blended for homogenation, to which either an uniform mixture of ingredients (c) and (d) is added or ingredients (c) and (d) are added independently. This process is carried out at room temperature or, depending on the nature of the starting materials, under heat.

When the thus obtained hair cosmetic composition in a gel state is applied to the hair, it acts as a hair treatment agent for keeping the hair healthy and beautiful.

The theoretical background which supports the effects of this invention is explained: Salts (a) of monoalkylphosphate form, even in a low concentration, a lamella liquid crystal, liposome (vesicle) or the like structure together with water and an alcohol. They are capable of stably capturing the volatile oil (c) and the non-volatile oil (d). The composition of this invention, consequently, is considered to form an O/W type gel structure.

Since the above-mentioned structure of the composition of this invention is readily destroyed when applied to the hair by hands or fingers, the extendibility is excellent, and the hair fibers can be covered by the composition smoothly without any difficulty. Furthermore, due to the O/W gel structure of the composition, hands and the hair never contact directly with the oil ingredients, which promises agreeable feeling upon use. What is more, after the hair is dried and the volatile oil is evaporated, the hair fibers are free of greasiness with very agreeable feeling.

Examples

This invention is now explained by way of examples, which however should not be construed as limiting the invention thereto.

Example 1:

Hair cosmetic compositions in a gel state formulated as shown in Table 1 were prepared, and their appearances were observed by the naked eye. Next, a panel consisting of 10 women used the compositions to compare the greasiness of them when received in hands, and feeling to the touch of the hair when applied to the hair and dried.

(1) Preparation:

Ingredients (1) - (3) were blended and dissolved at 60 °C. The temperature was returned to room temperature, and ingredients (4) - (6) were slowly added thereto while stirring.

(2) Evaluation Standard:

Extendibility:

- Easy to extend: O
- Difficult to extend: X

Greasiness:

- Free of greasiness: O
- Greasy and sticky: X

Feeling to touch of the hair after dried:

- Agreeable with innocence of greasiness: O
- Sticky and not agreeable: X

Table 1

| Ingredients (wt%) | Product 1 of Invention | Comparison product 1 | Comparison product 2 |
|---|---|---|---|
| (1) Arginine salt of 2-octalauryl phosphate | 0.5 | 0.5 | -- |
| (2) Water | 1 | 1 | 1 |
| (3) Glycerol | 5 | 5 | 5 |
| (4) Isoparaffin (Isozol 400) | 88.5 | -- | 89 |
| (5) Liquid paraffin (Hicol K-350) | -- | 88.5 | -- |
| (6) Dimethylpolysiloxane (KF-50, 300cs, by Shin'etsu Silicone) | 5 | 5 | 5 |
| Appearance | gel | gel | liquid (separation) |
| Extendibility | O | O | X |
| Greasiness | O | O | X |
| Feel of the hair when dried | O | X | X |

Example 2 Hair cosmetic composition in a gel state:

A composition was formulated using the following ingredients:

| | |
|---|---|
| (1) Arginine salt of 2-hexadecyl phosphate | 0.2% by weight |
| (2) Water | 2 |
| (3) Dimethylpolysiloxane (SE-76, by GE Co.) | 5 |

| | |
|---|---|
| (4) Glycerol | 5 |
| (5) Dimethylpolysiloxane of 4-membered ring (SH244, by Shin'etsu Kagaku) | balance |
| (6) Perfume, Colorant | suitable amount |
| Total: | 100.0 |

This composition presented a beautiful gel state appearance, was easy to extend, and the hair had excellent feel when the composition had dried on it.

Example 3 Hair cosmetic composition in a gel state:

A composition was formulated using the following ingredients:

| | |
|---|---|
| (1) Arginine salt of 2-hexadecyl phosphate | 0.2% by weight |
| (2) Water | 2 |
| (3) Dimethylpolysiloxane (n=3000 in formula (3)) | 5 |
| (4) Glycerol | 5 |
| (5) Isoparaffin (Isozol 400 by NISSEKI) | balance |
| (6) Perfume, Colorant | suitable amount |
| Total: | 100.0 |

This composition presented a beautiful gel state appearance, was easy to extend, and imparted excellent feel to the hair when the hair applied with this composition was dried.

**Claims**

**1.** A hair cosmetic composition in a gel state, which comprises the following ingredients (a), (b), (c), (d) and (e):
(a) a salt of a monoalkyl phosphate of formula (1):

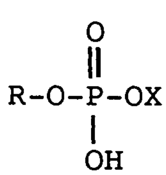

(1)

wherein R is a beta-branched alkyl having 8 to 36 carbon atoms, X is an alkali metal, basic amino acid or an organinc base;
(b) water;
(c) a volatile oil;
(d) a non-volatile oil; and
(e) an alcohol.

**2.** A hair cosmetic composition in a gel state according to Claim 1, wherein group R of the salt of

monoalkyl phosphate represented by formula (1) of ingredient (a) is represented by the following formula (2):

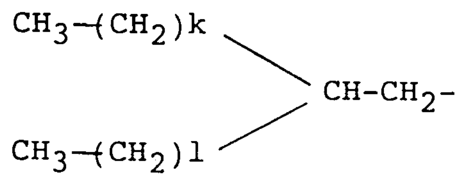

(2)

wherein k denotes a number of 2 - 18, 1 denotes a number of 2- 14 provided k plus 1 is from 4 to 32.

**3.** A hair cosmetic composition in a gel state according to either Claim 1 or Claim 2 wherein the volatile oil of ingredient (c) is a volatile linear silicone, volatile cyclic silicone or a volatile hydrocarbon.

**4.** A hair cosmetic composition in a gel state according to any of the preceding Claims wherein the non-volatile oil of ingredient (d) is selected from the group consisting of hydrocarbons, higher alcohols, higher fatty acids, higher fatty acid esters, oils and fats of animal or vegetable origin, cholesterol fatty acid esters, higher fatty acid glycerol esters, rosin derivatives and silicone oils; and said non-volatile oil has less than 50% volatility in 3 hours when permeated into a filter paper and allowed to stand at 30 ° C.

**5.** A hair cosmetic composition in a gel state according to any of the preceding Claims wherein the amounts of ingredients (a) to (e) are as follows:
(a): 0.05 - 5% by weight,
(b): 0.5 - 50% by weight,
(c): 50 - 98% by weight,
(d): 0.1 - 20% by weight,
(e): 2 - 50% by weight.

**6.** A hair cosmetic composition in a gel state according to any of the preceding Claims which has an O/W type gel structure.

**7.** A hair cosmetic composition in a gel state according to Claim 1, which is prepared by a process comprising the steps of blending to homogenisation ingredients (a), (b) and (c) and thereafter adding ingredients (c) and (d).